(19) Europäisches Patentamt
European Patent Office
Office européen des brevets

(11) EP 2 196 183 B1

(12) # FASCICULE DE BREVET EUROPEEN

(45) Date de publication et mention de la délivrance du brevet:
**16.11.2016 Bulletin 2016/46**

(21) Numéro de dépôt: **09178950.3**

(22) Date de dépôt: **11.12.2009**

(51) Int Cl.:
***A61K 8/49*** *(2006.01)*
***A61K 8/34*** *(2006.01)*
***A61K 8/44*** *(2006.01)*
***A61Q 5/10*** *(2006.01)*
***A61K 8/27*** *(2006.01)*
***A61K 8/368*** *(2006.01)*
***A61K 8/60*** *(2006.01)*
***A61K 8/365*** *(2006.01)*
***A61K 8/19*** *(2006.01)*
***A61K 8/22*** *(2006.01)*

(54) **Procédé de coloration à partir d'orthodiphénol et comprenant une étape d'essuyage, de séchage ou de non rincage**

Haarfärbeverfahren auf Basis eines Orthodiphenols mit einem Wisch-, Trocken- oder Nichtspülschritt

Dyeing method based on ortho-diphenol and comprising a wiping, drying or non rinsing stage

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **12.12.2008 FR 0858554**

(43) Date de publication de la demande:
**16.06.2010 Bulletin 2010/24**

(73) Titulaire: **L'Oréal**
**75008 Paris (FR)**

(72) Inventeur: **Guerin, Frédéric**
**75010, Paris (FR)**

(74) Mandataire: **Fevrier, Murielle Françoise E.**
**L'Oréal**
**Service DIPI**
**9 Rue Pierre Dreyfus**
**92110 Clichy (FR)**

(56) Documents cités:
**EP-A- 0 664 114**
**DE-A1- 19 959 480**
**DE-A1-102005 062 830**
**FR-A- 2 814 943**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 196 183 B1

Printed by Jouve, 75001 PARIS (FR)

## Description

L'invention a pour objet un procédé de coloration de fibres kératiniques par traitement desdites fibres avec i) au moins un dérivé d'orthodiphénol, ii) au moins un sel métallique, iii) au moins du peroxyde d'hydrogène ou système générateur de peroxyde et iv) au moins du (bi)carbonate ; faisant intervenir au moins une étape d'essuyage et/ou de séchage et/ou de non rinçage intermédiaire.

Il est connu d'obtenir des colorations dites « permanentes » avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. On sait également que l'on peut faire varier les nuances obtenues en associant ces bases d'oxydation à des coupleurs ou modificateurs de coloration, ces derniers étant choisis notamment parmi les méta-diamines aromatiques, les méta-aminophénols, les méta-diphénols et certains composés hétérocycliques tels que des composés indoliques. Ce procédé de coloration d'oxydation consiste à appliquer sur les fibres kératiniques, des bases ou un mélange de bases et de coupleurs avec du peroxyde d'hydrogène ($H_2O_2$ ou eau oxygénée) à titre d'agent oxydant, à laisser diffuser, puis à rincer les fibres. Les colorations qui en résultent sont permanentes, puissantes, et résistantes aux agents extérieurs, notamment à la lumière, aux intempéries, aux lavages, à la transpiration et aux frottements,

Cependant les colorations capillaires commerciales qui les contiennent peuvent présenter des inconvénients comme le tachage, les problèmes d'odeur, de confort et de dégradation des fibres kératiniques. C'est particulièrement le cas avec les colorations d'oxydation.

Dans le domaine de la coloration, il est également connu de colorer des matières kératiniques telles que les cheveux ou la peau à partir de composés orthodiphénols en présence d'un sel métallique notamment de Mn et/ou de Zn. En particulier les demandes de brevets FR 2 814 943, FR 2 814 945, FR 2 814 946, FR 2 814 947, proposent des compositions pour la coloration de la peau ou des fibres kératiniques, comprenant un précurseur de colorant qui contient au moins un orthodiphénol, des sels et oxydes de Mn et/ou Zn, des alcalins de type hydrogénocarbonate dans un ratio particulier Mn, Zn / hydrogénocarbonate et éventuellement une enzyme. Selon ces documents il est possible d'obtenir des colorations intenses en s'affranchissant du peroxyde d'hydrogène. Cependant les colorations obtenues sont insuffisamment intenses notamment dans le cas des fibres capillaires.

Il existe un besoin de développer des procédés de colorations qui permettent d'obtenir des colorations puissantes à partir d'orthodiphénols, notamment à partir d'extrait naturel riche en orthodiphénols, tout en limitant la décoloration des fibres kératiniques. En particulier, il existe un besoin d'obtenir des colorations moins agressives pour les cheveux et dans un même temps qui résistent aux agents extérieurs (lumière, intempéries, shampooings), qui soient tenaces et homogènes tout en restant puissantes et chromatiques.

Ce but est atteint par la présente invention qui a pour objet un procédé de coloration en plusieurs étapes consistant à:

a) dans un premier temps traiter lesdites fibres par :

i) un ou plusieurs dérivé(s) d'orthodiphénol(s),
ii) un ou plusieurs sel(s) métallique(s), et
iii) du peroxyde d'hydrogène ou un ou plusieurs système(s) générateur(s) de peroxyde d'hydrogène, les ingrédients i) à iii) étant appliqués sur lesdites fibres ensemble ou séparément ;

b) dans un deuxième temps lesdites fibres sont essuyées mécaniquement et/ou séchées et/ou non rincées préférentiellement lesdites fibres sont essuyées mécaniquement et/ou séchées et plus particulièrement lesdites fibres sont essuyées mécaniquement ; et

c) dans une troisième étape lesdites fibres sont traitées par iv) un ou plusieurs (bi)carbonate(s) plus particulièrement juste avant l'étape c) qui met en oeuvre l'ingrédient iv) les fibres sont essuyées mécaniquement

ou

a) dans un premier temps traiter lesdites fibres par :

i) un ou plusieurs dérivé(s) d'orthodiphénol(s), et
ii) un ou plusieurs sel(s) métallique(s),

b) dans un deuxième temps lesdites fibres sont essuyées mécaniquement et/ou séchées et/ou non rincées préférentiellement lesdites fibres sont essuyées mécaniquement et/ou séchées et plus particulièrement lesdites fibres

sont essuyées mécaniquement ; et

c) dans une troisième étape lesdites fibres sont traitées par iii) du peroxyde d'hydrogène ou un ou plusieurs système(s) générateur(s) de peroxyde d'hydrogène et iv) un ou plusieurs (bi)carbonate(s) ;

plus particulièrement juste avant l'étape c) qui met en oeuvre l'ingrédient iv) les fibres sont essuyées mécaniquement; étant entendu que les ingrédients i) et ii) et iii) et iv) peuvent être appliqués sur lesdites fibres ensemble ou séparément.

Le procédé selon l'invention présente l'avantage de colorer les fibres kératiniques humaines, avec des résultats de colorations puissantes, chromatiques, résistantes aux lavages, la transpiration, le sébum et à la lumière et de plus durables sans altération desdites fibres. De plus les colorations obtenues à partir du procédé donnent des couleurs homogènes de la racine à la pointe d'une fibre (faible sélectivité de coloration).

*i) dérivé(s) d'orthodiohénol(s) :*

Le premier ingrédient utilisé dans le procédé selon l'invention est i) un dérivé d'orthodiphénol.

Un mode particulier de l'invention concerne des dérivés d'orthodiphénols ou mélanges de composés comprenant au moins un cycle aromatique, de préférence un cycle benzénique, comportant au moins deux groupes hydroxy (OH) portés par deux atomes de carbone adjacents du cycle aromatique. Particulièrement le ou les dérivés d'orthodiphénols selon l'invention ne sont pas des dérivés autoxydables à motif indole. Plus particulièrement ils sont différents du 5,6-dihydroxyindole.

Le cycle aromatique peut être plus particulièrement un cycle aryle condensé ou hétéroaromatique condensé i.e. contenant éventuellement un ou plusieurs hétéroatomes, tel que le benzène, le naphtalène, le tétrahydronaphtalène, l'indane, l'indène, l'anthracène, le phénanthrène, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine, ledit cycle aromatique comportant au moins deux groupes hydroxy portés par deux atomes de carbone adjacents du cycle aromatique. Préférentiellement le cycle aromatique des dérivés orthodiphénols selon l'invention est un cycle benzénique.

Par cycle condensé on entend qu'au moins deux cycles saturés ou insaturés, hétérocycliques ou non, présentent une liaison commune i.e. qu'au moins un cycle soit accolé à un autre cycle.

Les orthodiphénols selon l'invention peuvent être salifiés ou non. Ils peuvent également se trouver sous forme d'aglygone (sans sucre liés) ou sous forme de composés glycosylés.

Plus particulièrement le dérivé d'orthodiphénol i) représente un composé de formule (I), ou l'un de ces oligomères, sous forme salifiée ou non :

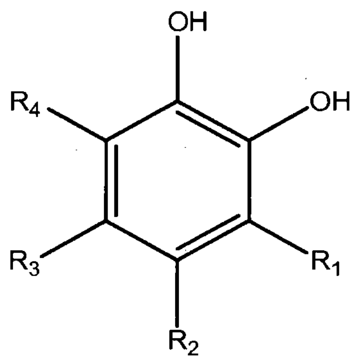

(I)

formule (I) dans laquelle les substituants :

- $R_1$ à $R_4$, identiques ou différents, représentent :
  - un atome d'hydrogène,
  - un atome halogène,
  - un radical hydroxy,
  - un radical carboxyle,
  - un radical carboxylate d'alkyle ou alcoxycarbonyle,
  - un radical amino éventuellement substitué,
  - un radical alkyle linéaire ou ramifié éventuellement substitué,
  - un radical alcényle linéaire ou ramifié éventuellement substitué,

- un radical cycloalkyle éventuellement substitué,
- un radical alcoxy,
- un radical alcoxyalkyle,
- un radical alcoxyaryle, le groupe aryle pouvant être éventuellement substitué,
- un radical aryle,
- un radical aryle subsitué
- un radical hétérocyclique, saturé ou non, porteur ou non d'une charge cationique ou anionique, éventuellement substitué et/ou éventuellement condensé avec un cycle aromatique de préférence benzénique, ledit cycle aromatique étant éventuellement substitué particulièrement par un ou plusieurs groupements hydroxy ou glycosyloxy,
- un radical contenant un ou plusieurs atomes de silicium,

• où deux des substituants portés par deux atome de carbone adjacents $R_1$ - $R_2$, $R_2$- $R_3$ ou $R_3$ - $R_4$ forment conjointement avec les atomes de carbone les portant un cycle saturé ou insaturé, aromatique ou non, contenant éventuellement un ou plusieurs hétéroatomes et éventuellement condensé avec un ou plusieurs cycles saturés ou insaturés contenant éventuellement un ou plusieurs hétéroatomes. Particulièrement $R_1$ à $R_4$ forment conjointement de un à quatre cycles.

Un mode de réalisation particulier de l'invention concerne des dérivés d'orthodiphénols de formule (I) dont deux substituants adjacents $R_1$ - $R_2$, $R_2$ - $R_3$ ou $R_3$ - $R_4$ ne peuvent former avec les atomes de carbone qui les portent un radical pyrrolyle. Plus particulièrement $R_2$ et $R_3$ ne peuvent former un radical pyrrolyle condensé au cycle benzénique portant les deux hydroxy.

Les cycles saturés ou insaturés, éventuellement condensés, peuvent être aussi éventuellement substitués.

Les radicaux alkyles sont des radicaux hydrocarbonés, saturés, linéaires ou ramifiés, généralement en $C_1$-$C_{20}$, particulièrement en $C_1$-$C_{10}$, de préférence les radicaux alkyles en $C_1$-$C_6$, tels que méthyle, éthyle, propyle, butyle, pentyle et hexyle.

Les radicaux alcényles sont des radicaux hydrocarbonés, linéaires ou ramifiés, insaturés en $C_2$-$C_{20}$; de préférence comprenant au moins une double liaison tels que éthylène, propylène, butylène, pentylène, méthyl-2-propylène et décylène.

Les radicaux aryles sont des radicaux carbonés mono ou polycycliques, condensés ou non, comprenant préférentiellement de 6 à 30 atomes de carbone et dont au moins un cycle est aromatique ; préférentiellement choisis parmi le radical aryle est un phényle, biphényle, naphtyle, indényle, anthracényle, et tétrahydronaphtyle.

Les radicaux alcoxy sont des radicaux alkyle-oxy avec alkyle tel que défini précédemment, de préférence en $C_1$-$C_{10}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxy, amine et cétone.

Les radicaux alkyles ou alcényles lorsqu'ils sont éventuellement substitués, peuvent être substitués par au moins un substituant porté par au moins un atome de carbone, choisi parmi :

- un atome d'halogène;
- un groupement hydroxy ;
- un radical alcoxy en $C_1$-$C_2$ ;
- un radical alcoxycarbonyle en $C_1$-$C_{10}$;
- un radical (poly)-hydroxyalcoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  * un groupement hydroxy,
  * un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au

moins un autre hétéroatome différent ou non de l'azote,

* un groupement ammonium quaternaire $-N^+R'R''R'''$, M- pour lequel R', R", R"', identiques ou différents représentent un atome d'hydrogène, ou un groupement alkyle en $C_1$-$C_4$; et M- représente le contre-ion de l'acide organique, minéral ou de l'halogénure correspondant,

* ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- un groupement cyano ;
- un groupement nitro ;
- un groupement carboxy ou glycosylcarbonyle ;
- un groupement phénylcarbonyloxy éventuellement substitué par un ou plusieurs groupements hydroxy ;
- un groupement glycosyloxy ; et
- un groupement phényle éventuellement substitué par un ou plusieurs groupements hydroxy.

Les radicaux aryles ou hétérocycliques ou la partie aryle ou hétérocycliques des radicaux lorsqu'ils sont éventuellement substitués peuvent être substitués par au moins un substituant porté par au moins un atome de carbone, choisi parmi :

- un radical alkyle en $C_1$-$C_{10}$, de préférence en $C_1$-$C_8$, éventuellement substitué par un ou plusieurs radicaux choisis parmi les radicaux hydroxyle, alcoxy en $C_1$-$C_2$, (poly)-hydroxyalcoxy en $C_2$-$C_4$, acylamino, amino substitué par deux radicaux alkyle, identiques ou différents, en $C_1$-$C_4$, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
- un atome d'halogène;
- un groupement hydroxy ;
- un radical alcoxy en $C_1$-$C_2$ ;
- un radical alcoxycarbonyle en $C_1$-$C_{10}$ ;
- un radical (poly)-hydroxyalcoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

* un groupement hydroxy,

* un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,

* un groupement ammonium quaternaire $-N^+R'R''R'''$, M- pour lequel R', R", R"', identiques ou différents représentent un atome d'hydrogène, ou un groupement alkyle en $C_1$-$C_4$; et M- représente le contre-ion de l'acide organique, minéral ou de l'halogénure correspondant,

* ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$

éventuellement porteur d'au moins un groupement hydroxy et le radical R' est un radical alkyle en $C_1$-$C_2$ ; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino (R'$SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,

- un radical carboxylique sous forme acide ou salifiée (de préférence avec un métal alcalin ou un ammonium, substitué ou non) ;
- un groupement cyano ;
- un groupement nitro ;
- un groupement polyhalogénoalkyle, préférentiellement le trifluorométhyle ;
- un groupement carboxy ou glycosylcarbonyle ;
- un groupement phénylcarbonyloxy éventuellement substitué par un ou plusieurs groupements hydroxy ;
- un groupement glycosyloxy ; et
- un groupement phényle éventuellement substitué par un ou plusieurs groupements hydroxy.

Par radical glycosyle on entend un radical issu d'un mono ou polysacharride.

Les radicaux contenant un ou plusieurs atomes de silicium sont de préférence des radicaux poly-diméthylsiloxane, poly-diphénylsiloxane, poly-diméthylphénylsiloxane, stéaroxy-diméthicone.

Les radicaux hétérocycliques sont en général des radicaux comprenant dans au moins un cycle un ou plusieurs hétéroatomes choisis parmi O, N et S, de préférence O ou N, éventuellement substitués par notamment un ou plusieurs groupes alkyles, alcoxy, acide carboxylique, hydroxy, amine ou cétone. Ces cycles peuvent contenir un ou plusieurs groupements oxo sur les atomes de carbone de l'hétérocycle.

Parmi les radicaux hétérocycliques utilisables, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les orthodiphénols utiles dans le procédé de l'invention peuvent être naturels ou synthétiques. Parmi les orthodiphénols naturels on inclut les composés qui peuvent être présents dans la nature et qui sont reproduits par (hémi)synthèse chimique.

Les sels des orthodiphénols de l'invention peuvent être des sels d'acides ou de bases. Les acides peuvent être minéraux ou organiques. De préférence l'acide est l'acide chlorhydrique qui conduit aux chlorures.

Les bases peuvent être minérales ou organiques. Particulièrement les bases sont des hydroxydes alcalins tels que la soude qui conduit à des sels de sodium.

Selon un mode de réalisation particulier de l'invention, la composition comprend comme ingrédient i) un ou plusieurs dérivé(s) d'orthodiphénol(s) synthétique(s) qui n'existe pas dans la nature.

Selon un autre mode de réalisation préféré de l'invention, le procédé de coloration des fibres kératiniques met en oeuvre comme ingrédient i) un ou plusieurs dérivé(s) d'orthodiphénol(s) naturel(s).

Plus particulièrement, les orthodiphénols utilisables dans le procédé de l'invention selon i) sont en particulier :

- les flavanols comme la catéchine et le gallate d'épichatéchine,
- les flavonols comme la quercétine,
- les anthocyanidines comme la cyanidine, la delphinidine, la pétunidine,
- les anthocyanines ou les anthocyanes comme la myrtilline ,
- les orthohydroxybenzoates, par exemple les sels d'acide gallique,
- les flavones comme la lutéoline,
- les hydroxystilbènes, par exemple le tétrahydroxy-3,3',4,5'-stilbène, éventuellement oxylés (par exemple glucosylés),
- la 3,4-dihydroxyphénylalanine et ses dérivés,
- la 2,3-dihydroxyphénylalanine et ses dérivés,
- la 4,5-dihydroxyphénylalanine et ses dérivés,
- les dihydroxycinnamates tels que l'acide caféique et l'acide chlorogénique,
- les orthopolyhydroxycoumarines,
- les orthopolyhydroxyisocoumarines,
- les orthopolyhydroxycoumarones,

- les orthopolyhydroxyisocoumarones,
- les orthopolyhydroxychalcones,
- les orthopolyhydroxychromones,
- les orthopolyhydroxyquinones,
- les orthopolyhydroxyxanthones,
- le 1,2 dihydroxybenzène et ses dérivés,
- le 1,2,4 trihydroxybenzène et ses dérivés,
- le 1,2,3 trihydroxybenzène et ses dérivés,
- le 2,4,5 trihydroxytoluène et ses dérivés,
- les proanthocyanidines et notamment les proanthocyanidines A1, A2, B1, B2, B3 et C1,
- les proanthocyanines,
- l'acide tannique,
- l'acide ellagique,
- et les mélanges des composés précédents.

Lorsque les précurseurs de coloration présentent des formes D et L, les deux formes peuvent être utilisées dans les compositions selon l'invention, ainsi que les racémiques.

Selon un mode de réalisation les orthodiphénols naturels sont issus d'extraits d'animaux, de bactéries, de champignons, d'algues, de plantes utilisés dans leur totalité ou partiellement. En particulier concernant les plantes les extraits sont issus de plante ou de partie de plantes telles que les fruits dont les agrumes, les légumes, les arbres, les arbustes. On peut aussi utiliser des mélanges de ces extraits, riches en othodiphénols tels que définis précédemment.

De préférence le ou les orthodiphénols naturels de l'invention sont issus d'extraits de plantes ou de parties de plantes

Au sens de l'invention on assimilera lesdits extraits dans leur ensemble en tant que composé i).

Les extraits sont obtenus par extraction de diverses parties de plantes telles que par exemple la racine, le bois, l'écorce, la feuille, la fleur, le fruit, le pépin, la gousse, la pelure.

Parmi les extraits de plantes, on peut citer les extraits de feuilles de thé, de rose, les extraits de feuilles de romarin ou les extraits de feuilles de maté.

Parmi les extraits de fruits, on peut citer les extraits de pomme, de raisin (en particulier de pépins de raisin), ou les extraits de fèves et/ou cabosses de cacaoyer.

Parmi les extraits de légumes, on peut citer les extraits de pomme de terre, de pelures d'oignon.

Parmi les extraits de bois d'arbres, on peut citer les extraits d'écorce de pin, les extraits de bois de campêche.

On peut également utiliser des mélanges d'extraits végétaux.

Selon un mode de réalisation particulier de l'invention le ou les dérivés d'orthodiphénols sont des extraits naturels, riches en orthodiphénols. Selon un mode préféré, le ou les dérivés d'orthodiphénols sont uniquement des extraits naturels.

Les extraits naturels selon l'invention peuvent se présenter sous forme de poudres ou de liquides. De préférence les extraits de l'invention se présentent sous forme des poudres.

Selon l'invention, le ou les dérivé(s) d'orthodiphénol(s) synthétique(s), naturel(s), et/ou le ou les extrait(s) naturel(s) utilisé(s) comme ingrédient i) dans une ou plusieurs composition(s) utile(s) dans le procédé selon l'invention représente(nt) de préférence, de 0.001% à 20% en poids du poids total de la ou des compositions contenant le ou les orthodiphénols ou le ou les extraits.

En ce qui concerne les orthodiphénols purs, la teneur dans la ou les compositions les contenant est comprise de préférence entre 0,001 et 5 % en poids de chacune de ces compositions.

En ce qui concerne les extraits, la teneur dans la ou les compositions contenant les extraits tels quels est comprise de préférence entre 0,5 et 20 % en poids de chacune de ces compositions.

*ii) sel(s) métallique(s).*

Le procédé de l'invention utilise un ou plusieurs ingrédient(s) ii) qui sont des sel(s) métallique(s).

Particulièrement les sels métalliques sont choisis parmi les sels de Manganèse (Mn) et de Zinc (Zn).

Au sens de la présente invention on entend par sels, les oxydes de ces métaux et les sels proprement dits issus notamment de l'action d'un acide sur un métal. De préférence les sels ne sont pas des oxydes. Parmi les sels on peut citer les halogénures tels que les chlorures, fluorures et iodures ; les sulfates, les phosphates ; les nitrates ; les perchlorates et les sels d'acides carboxyliques et les complexes polymériques pouvant supporter lesdits sels, ainsi que leurs mélanges.

Plus particulièrement le sel de manganèse est différent du carbonate de manganèse, de l'hydrogénocarbonate de manganèse ou du dihydrogénocarbonate de manganèse.

Les sels d'acides carboxyliques utilisables dans l'invention incluent également des sels d'acides carboxyliques hydroxylés tels que le gluconate.

A titre d'exemple de complexes polymériques pouvant supporter lesdits sels on peut citer pyrrolidone carboxylate de manganèse.

A titre d'exemple, on peut citer le chlorure de manganèse, le fluorure de Mn, l'acétate de Mn tétrahydraté, le lactate de Mn trihydraté, le phosphate de Mn, l'iodure de Mn, le nitrate de Mn trihydraté, le bromure de Mn, le perchlorate de Mn tétrahydraté, le sulfate de Mn monohydraté et le gluconate de manganèse. Les sels utilisés avantageusement sont le gluconate de manganèse et le chlorure de manganèse.

Parmi les sels de zinc on peut citer le sulfate de zinc, le gluconate de zinc, le chlorure de zinc, le lactate de zinc, l'acétate de zinc, le glycinate de zinc et l'aspartate de zinc.

Les sels de manganèse et de zinc peuvent être introduits sous forme solide dans les compositions ou bien provenir d'une eau naturelle, minérale ou thermale, riche en ces ions ou encore d'eau de mer (mer morte notamment). Ils peuvent aussi provenir de composés minéraux comme les terres, les ocres comme les argiles (argile verte par exemple) ou même d'extrait végétal les contenant (cf. par exemple brevet le document FR 2 814 943).

Particulièrement les sels métalliques de l'invention sont de degrés d'oxydation 2 tels que le Mn(II) et le Zn(II).

Selon un mode de réalisation préféré de l'invention, le ou les sels métalliques utilisés représentent de 0.001% à 10% en poids environ du poids total de la ou des composition(s) contenant ce ou ces sels métalliques et encore plus préférentiellement de 0,05% à 0,1% en poids environ.

*iii) peroxyde d'hydrogène ou système(s) générateur(s) de peroxyde d'hydrogène*

Dans le cadre de la présente invention, le troisième ingrédient est du peroxyde d'hydrogène ou un ou plusieurs système(s) générateur(s) de peroxyde d'hydrogène tel(s) que :

a) le peroxyde d'urée ;

b) les complexes polymériques pouvant libérer du peroxyde d'hydrogène tels que le polyvinylpyrrolidone/$H_2O_2$ en particulier se présentant sous forme de poudres et les autres complexes polymériques décrits dans US 5,008,093 ; US 3,376,110 ; US 5,183,901 ;

c) les oxydases produisant du peroxyde d'hydrogène en présence d'un substrat adéquat (par exemple le glucose dans le cas de glucose oxydase ou l'acide urique avec l'uricase) ;

d) les peroxydes de métaux générant dans l'eau du peroxyde d'hydrogène comme le peroxyde de calcium, peroxyde de magnésium;

e) les perborates ; ou

f) les percarborates.

Selon un mode de réalisation préféré de l'invention la composition contient un ou plusieurs système(s) générateur(s) de peroxyde d'hydrogène choisi(s) parmi a) le peroxyde d'urée, b) les complexes polymériques pouvant libérer du peroxyde d'hydrogène choisis parmi le polyvinylpyrrolidone/$H_2O_2$; c) les oxydases ; e) les pérborates et f) le percarborates.

Particulièrement le troisième constituant est du peroxyde d'hydrogène.

Par ailleurs la ou les compositions comprenant le peroxyde d'hydrogène ou le ou les générateur(s) de peroxyde d'hydrogène peut ou peuvent également renfermer divers adjuvants utilisés classiquement dans les compositions pour la teinture des cheveux et tels que définis ci-après au point vii).

Selon un mode particulier de l'invention, le peroxyde d'hydrogène ou le ou les système(s) générateur(s) de peroxyde d'hydrogène utilisé(s) représente(nt) de préférence, de 0.001% à 12% en poids exprimé en peroxyde d'hydrogène par rapport au poids total de la composition ou des compositions le ou les contenant et encore plus préférentiellement de 0,2% à 2,7% en poids.

*iv) (bi)carbonate(s)*

Dans le cadre de la présente invention, le quatrième ingrédient est choisi parmi les (bi)carbonates.

Par (bi)carbonates est sous entendu :

a) les carbonates de métal alcalins ($Mét_2^+$, $CO_3^{2-}$), de métal alcalino-terreux ($Mét'^{2+}$, $CO_3^{2-}$) d'ammonium ($(R''_4N^+)_2,CO_3^{2-}$) ou de phosphonium ($(R''_4P^+)_2,CO_3^{2-}$ avec Mét' représentant un métal alcalino-terreux et Mét représentant un métal alcalin, et R", identiques ou différents, représentent un atome d'hydrogène, un groupement ($C_1$-$C_6$)alkyle éventuellement substitué tel que hydroxyéthyle), et

b) les bicarbonates, également appelés hydrogénocarbonates, de formules suivantes :

➢ $R'^{+}$, $HCO_3^-$ avec R' représentant un atome d'hydrogène, un métal alcalin, un groupement ammonium $R''_4N^+$- ou phosphonium $R''_4P^+$- où R", identiques ou différents, représentent un atome d'hydrogène, un groupement $(C_1-C_6)$alkyle éventuellement substitué tel qu'hydroxyéthyle et lorsque R' représente un atome d'hydrogène l'hydrogénocarbonate est alors appelé dihydrogénocarbonate ($CO_2$, $H_2O$) ; et

➢ $Mét'^{2+}(HCO_3^-)_2$ avec Mét' représentant un métal alcalino-terreux.

Plus particulièrement, le quatrième ingrédient est choisi parmi les (bi)carbonates de métal alcalin ou alcalino-terreux ; préférentiellement les (bi)carbonates de métal alcalin.

On peut citer les carbonates ou hydrogénocarbonates de Na, K, Mg, Ca et leurs mélanges, et en particulier l'hydrogénocarbonate de Na. Ces hydrogénocarbonates peuvent provenir d'une eau naturelle, par exemple eau de source du bassin de Vichy, de La Roche Posay, eau de Badoit (cf. brevet par exemple le document FR 2 814 943). Particulièrement on peut citer le carbonate de sodium [497-19-8] = $Na_2CO_3$, l'hydrogénocarbonate de sodium ou bicarbonate de soude [144-55-8] = $NaHCO_3$, et le dihydrogénocarbonate de sodium = $Na(HCO_3)_2$.

Selon l'invention, le ou les agents (bi)carbonates utilisés représentent de préférence, de 0.001% à 10% en poids du poids total de la ou des compositions contenant le ou les agents (bi)carbonates et encore plus préférentiellement de 0,005% à 5% en poids.

*v) étape(s) d'essuyage mécanique et/ou séchage et/ou de non rinçage:*

Le procédé de coloration des fibres kératiniques selon l'invention comprend au moins une étape intermédiaire d'essuyage mécanique des fibres et/ou de séchage et/ou non rincées.

Les étapes d'essuyage mécanique et de séchage intermédiaire sont également appelées « non rincé maitrisé » pour se différencier du « rinçage classique à grande eau » et du « non rincé ».

Par essuyage mécanique des fibres on entend le frottement d'un objet absorbant sur les fibres et le retrait physique par l'objet absorbant du surplus d'ingrédient(s) n'ayant pas pénétré dans les fibres. L'objet absorbant peut être une pièce de tissu comme une serviette particulièrement éponge, un torchon, du papier absorbant tel que de l'essuie-tout.

Selon un procédé particulièrement avantageux de l'invention, l'essuyage mécanique est effectué sans séchage totale de la fibre, laissant la fibre humide.

Par séchage on entend l'action d'évaporer des solvants organiques et/ou de l'eau se trouvant dans une ou plusieurs compositions utilisées dans le procédé de l'invention, comprenant ou non un ou plusieurs ingrédients i à iv) tels que définis précédemment. Le séchage peut se faire par source thermique (convection, conduction ou rayonnement) en envoyant par exemple un courant gazeux chaud tel que l'air nécessaire à l'évaporation du ou des solvants. A titre de source thermique on peut citer un sèche-cheveux, de casques à cheveux, d'un fer à lisser les cheveux, d'un dispensateur de rayons infrarouges et d'autres appareils chauffants classiques.

*vi) l'eau:*

Selon un mode de réalisation de l'invention, de l'eau est de préférence incluse dans le procédé de l'invention. Elle peut provenir de l'humidification des fibres kératiniques et/ou de la ou des compositions comprenant les composés i) à iv) tels que définis précédemment ou d'une ou plusieurs autres compositions. De préférence l'eau provient au moins d'une composition comprenant au moins un composé choisi parmi i) à iv) tels que définis précédemment.

*vii) compositions cosmétiques:*

Les ingrédients i) à iv) peuvent se trouver seuls ou dans une composition cosmétique. Les compositions cosmétiques utilisées dans le procédé selon l'invention sont cosmétiquement acceptable i.e. elles comprennent un support de coloration qui contient généralement de l'eau ou un mélange d'eau et d'un ou plusieurs solvants organiques ou un mélange de solvants organiques.

Par solvant organique, on entend une substance organique capable de dissoudre ou disperser une autre substance sans la modifier chimiquement.

*Les Solvants organiques:*

A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de

propylèneglycol, le monoéthyléther et le monométhyléther du diéthylèneglycol, l'hexylène glycol, ainsi que les alcools aromatiques comme l'alcool benzylique ou le phénoxyéthanol.

Les solvants organiques sont présents dans des proportions de préférence comprises entre 1 et 40 % en poids environ par rapport au poids total de la composition tinctoriale, et encore plus préférentiellement entre 5 et 30 % en poids environ.

*Les Adjuvants:*

La ou les compositions du procédé de coloration conforme à l'invention peut également renfermer divers adjuvants utilisés classiquement dans les compositions pour la teinture des cheveux, tels que des agents tensio-actifs anioniques, non-ioniques, cationiques, amphotères, zwitterioniques ou leurs mélanges, des polymères anioniques autres que des tensioactifs, des polymères cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiant.

Lesdits adjuvants sont choisis de préférence parmi des agents tensioactifs tels que des tensioactifs anioniques, non ioniques ou leurs mélanges et des agents épaississants minéraux ou organiques.

Les adjuvants ci-dessus sont en général présents en quantité comprise pour chacun d'eux entre 0,01 et 40 % en poids par rapport au poids de la composition ou des compositions comprenant les ingrédients i) à iv) tels que définis précédemment, de préférence entre 0,1 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition ou aux composition(s) utiles dans le procédé de coloration conforme à l'invention ne soient pas, ou substantiellement pas, altérées par la ou les adjonctions envisagées.

*Les Colorants additionnels:*

Le procédé mettant en oeuvre les ingrédients i) à iv) tels que définis précédemment peut en outre mettre en oeuvre ou comprendre un ou plusieurs colorants directs additionnels. Ces colorants directs sont par exemple choisis parmi ceux classiquement utilisés en coloration directe, et parmi lesquels on peut citer tous les colorants aromatiques et/ou non aromatiques d'utilisation courante tels que les colorants directs nitrés benzéniques neutres, acides ou cationiques, les colorants directs azoïques neutres, acides ou cationiques, les colorants directs naturels autres que les orthodiphénols, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les colorants fluorescents. Tous ces colorants additionnels sont différents des dérivés d'orthodiphénols selon l'invention.

Parmi les colorants directs naturels, on peut citer la lawsone, la juglone, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Le ou les colorants directs additionnels utilisés dans la ou les compositions comprenant les ingrédients i) à iv) utilisés dans le procédé selon l'invention, représentent de préférence, de 0.001% à 10% en poids environ du poids total de la ou des compositions les contenant et encore plus préférentiellement de 0.05% à 5% en poids environ.

Les compositions du procédé mettant en oeuvre les ingrédients i) à iv) tels que définis précédemment peuvent également mettre en oeuvre une ou plusieurs bases d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

La ou les bases d'oxydation présentes dans la ou les compositions sont en général présentes chacune en quantité comprise entre 0,001 à 10 % en poids du poids total de la ou des compositions correspondantes.

La ou les composition(s) cosmétique(s) utilisée(s) dans le procédé de l'invention peuvent se présenter sous des formes galéniques diverses, telles qu'une poudre, une lotion, une mousse, une crème, un gel ou sous tout autre forme appropriée pour réaliser une teinture des fibres kératiniques. Elles peuvent également être conditionnées en flacon pompe sans propulseur ou sous pression en flacon aérosol en présence d'un agent propulseur et former une

mousse.

*pH de la ou des composition(s) du procédé*

Selon un mode particulier de l'invention le pH de la ou des compositions du procédé contenant le ou les (bi)carbonates est supérieur à 7 et de préférence compris entre 8 et 12. Particulièrement compris entre 8 et 10.

Si la ou les compositions, ne contiennent pas de (bi)carbonates, le pH de la ou des compositions contenant le peroxyde d'hydrogène ou un système générateur de peroxyde d'hydrogène, est de préférence inférieur à 7, plus particulièrement compris entre 1 et 5.

De préférence la ou les compositions contenant le ou les orthodiphénols de l'invention et ne contenant pas de (bi)carbonates sont à pH intérieur à 7 et de préférence comprise entre 3 et 6,5.

Selon un mode particulier du procédé de l'invention les compositions contenant le ou les sels métalliques et ne contenant de (bi)carbonates sont à pH inférieur à 7 et de préférence comprise entre 3 et 6,5.

Le pH de ces compositions peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants des compositions utilisées dans l'invention, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Une variante avantageuse est d'ajouter un agent alcalinisant à la ou les compositions du procédé de coloration contenant le ou les (bi)carbonates. Plus particulièrement, cet agent alcalin est choisi parmi l'ammoniaque, les carbonates alcalins, les alcanolamines telles que les mono-, di- et triéthanolamines ainsi que leurs dérivés, les hydroxydes de sodium ou de potassium et les composés de formule (II) suivante :

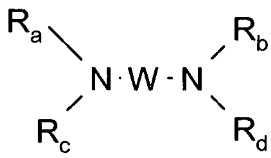
(II)

dans laquelle W est un reste propylène éventuellement substitué par un groupement hydroxy ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

*viii) Procédé de coloration en plusieurs étapes*

Selon le premier mode de réalisation de l'invention le procédé de coloration des fibres kératiniques consiste à :

a) dans un premier temps traiter lesdites fibres par :

i) un ou plusieurs dérivé(s) d'orthodiphénol(s),
ii) un ou plusieurs sel(s) métallique(s), de préférence choisis parmi les sels de Mn et de Zn, et
iii) du peroxyde d'hydrogène ou un ou plusieurs système(s) générateur(s) de peroxyde d'hydrogène,

les ingrédients i) à iii) étant appliqués sur lesdites fibres ensemble ou séparément ;
c) dans un deuxième temps lesdites fibres sont essuyées mécaniquement et/ou séchées et/ou non rincées préférentiellement lesdites fibres sont essuyées mécaniquement et/ou séchées ; et
d) dans une troisième étape lesdites fibres sont traitées par iv) un ou plusieurs (bi)carbonate(s)

Selon le second mode de réalisation de l'invention le procédé de coloration des fibres kératiniques consiste à. :

a) dans un premier temps traiter lesdites fibres par :

i) un ou plusieurs dérivé(s) d'orthodiphénol(s), et
ii) un ou plusieurs sel(s) métallique(s), de préférence choisis parmi les sels de Mn et de Zn,

b) dans un deuxième temps lesdites fibres sont essuyées mécaniquement et/ou séchées et/ou non rincées préférentiellement lesdites fibres sont essuyées mécaniquement et/ou séchées ; et

c) dans une troisième étape lesdites fibres sont traitées par iii) du peroxyde d'hydrogène ou un ou plusieurs système(s) générateur(s) de peroxyde d'hydrogène et iv) un ou plusieurs (bi)carbonate(s) ;

étant entendu que les ingrédients i) et ii) et iii) et iv) peuvent être appliqués sur lesdites fibres ensemble ou séparément.

Selon un procédé particulièrement préféré de l'invention juste avant l'étape qui met en oeuvre l'ingrédient iv) les fibres sont a) essuyées mécaniquement.

Les fibres kératiniques peuvent être ou non préalablement humidifiées.

Dans la deuxième étape lesdites fibres peuvent être essuyée mécaniquement une seule fois ou une succession de fois jusqu'à disparition de l'excédent d'ingrédients i) et ii) ou d'ingrédients i), ii) et iii).

Dans la deuxième étape si le moyen de séchage est thermique par exemple un sèche-cheveux, casque à cheveux ou fer à lisser ou à l'air libre, alors les fibres sont séchées jusqu'à disparition visuelle de l'aspect mouillé des cheveux. Particulièrement les fibres kératiniques sont séchées à l'aide d'un fer à lisser ou d'un sèche-cheveux.

Le temps de pause entre les étapes d'application des ingrédients i), ii), iii), iv) est fixé entre 3 et 120 minutes, préférentiellement entre 10 et 60 minutes et plus particulièrement entre 15 et 45 minutes.

Un ou plusieurs essuyages et/ou séchages, successifs ou non, peuvent être envisagés entre les applications d'ingrédient i) et ii), ou entre chacune des applications d'ingrédients i), ii) et iii) ou entre chacune des applications d'ingrédient i), ii), iii) et iv) ou entre les applications de mélange i)+ii) et de mélange iii)+iv) ou entre l'application de mélange i)+ii), et d'ingrédients iii) et iv).

Particulièrement l'étape d'essuyage et/ou de séchage est réalisée après application du mélange d'ingrédients i) + ii) + iii) tels que définis précédemment et avant l'application de l'ingrédient iv).

Plus particulièrement le procédé fait intervenir une ou plusieurs étapes successives d'essuyage après l'application du mélange de i) + ii) + iii). Dans ce cas la succession d'essuyage est réalisée jusqu'à disparition visuelle de l'excédant dudit mélange et séchage des fibres.

Le temps de pause après application de chacun des ingrédients ou de mélange est généralement fixé entre 3 et 120 minutes et préférentiellement entre 10 et 60 minutes et plus préférentiellement entre 15 et 45 minutes.

Quelque soit le mode d'application, la température d'application des ingrédients i) à iv) est généralement comprise entre la température ambiante (15 à 25°C) et 80°C et plus particulièrement entre 15 et 45°C. Ainsi, on peut, avantageusement, après application de la composition selon l'invention, soumettre la chevelure à un traitement thermique par chauffage à une température comprise entre 30 et 60°C.

Lorsque dans le procédé il est utilisé un moyen de chauffage thermique un fer chauffant sa température comprise entre 60 et 220°C et de préférence entre 120 et 200°C.

Un mode particulier de l'invention concerne un procédé de coloration qui est réalisé à température ambiante (25 °C) pour l'application des ingrédients i) à iv).

Dans tous les modes particuliers et variantes des procédés précédemment décrits les compositions évoquées sont des compositions prêtes à l'emploi qui peuvent résulter du mélange extemporané de deux ou plusieurs compositions et notamment de compositions présentes dans des kits de teintures.

## I) EXEMPLES DE COLORATION

La composition suivante a été préparée :

| Composition A | A |
|---|---|
| Catéchine | 5g |
| Héxylène glycol | 5g |
| Lauryl ether sulfate de sodium (70% en MA dans l'eau) | 3,75g |
| Gluconate de manganèse (soit 0,01% en poids d'équivalent métal $Mn^{2+}$) | 0,081 g |
| Peroxyde d'hydrogène | 1,2g |
| Acide citrique ou hydroxyde de sodium | qsp pH 5 |
| Eau déminéralisée | qsp 100g |

La composition **A** est appliquée sur des mèches de cheveux secs à 90% de blancs naturels et à 90% de blancs permanentés avec un rapport de bain de 5 g de formule pour 1g de cheveux. On laisse ensuite poser pendant 30 minutes à une température de 50°C.

A l'issu, les cheveux imprégnés de la première composition sont essuyés à l'aide d'une serviette absorbante

pour enlever l'excédant de formule et éventuellement séché ou pas.

| Composition B | B |
|---|---|
| Bicarbonate de sodium $NaHCO_3$ | 5g |
| Carbomer | 1g |
| Monoéthanolamine | qsp pH 9 |
| Eau déminéralisée | qsp 100g |

La composition **B** est ensuite appliquée sur les cheveux avec un rapport de bain de 4g pour 1 g de mèche ; le temps de pose est de 10 minutes à température ambiante. Au bout de quelques minutes une coloration très intense apparaît.

Les cheveux sont ensuite rincés à l'eau, lavés avec un shampooing classique et séchés au casque.

La coloration est très tenace aux lavages et à la lumière.

**Résultats colorimétriques :**

La coloration des cheveux est évaluée visuellement et lue au spectrocolorimètre Minolta (CM3600d, illuminant D65, angle 10°, valeurs SCI) pour les mesures colorimétriques L*, a*, b*.

Dans ce système L* a* b*, L* représente l'intensité de la couleur, a* indique l'axe de couleur vert/rouge et b* l'axe de couleur bleu/jaune. Plus la valeur de L est faible, plus la couleur est foncée ou très intense. Plus la valeur de a* est élevée plus la nuance est rouge et plus la valeur de b* est élevée plus la nuance est jaune.

a) La variation de coloration entre les mèches colorées de cheveux blancs naturels/permanentés non traités (témoin) et après traitement sont définis par (ΔE*) selon l'équation suivante :

$$\Delta E^* = \sqrt{(L^* - L_o^*)^2 + (a^* - a_o^*)^2 + (b^* - b_o^*)^2}$$

Dans cette équation, L*, a* et b* représentent les valeurs mesurées après coloration des cheveux naturels/permanentés à 90% de blancs et $L^*_0$, $a_0^*$ et $b_0^*$ représentent les valeurs mesurées des cheveux naturels/permanentés à 90% non traités.

Plus la valeur de ΔE* est importante, plus la différence de couleur entre les mèches témoins et les mèches colorées est importante.

b) La variation de coloration entre les mèches blanc permanentées et de cheveux blancs naturels correspondant à la différence de sélectivité ($\Delta E_{sélec}$) est donnée par l'équation suivante :

$$\Delta E_{sélec} = \sqrt{(L - L_o)^2 + (a - a_o)^2 + (b - b_o)^2}$$

Dans cette équation, L, a et b représentent les valeurs mesurées après coloration des cheveux permanentés à 90% de blancs et $L_0$, $a_0$ et $b_0$ représentent les valeurs mesurées après coloration des cheveux naturels à 90%.

Plus la valeur de $\Delta E_{sélec}$ est faible, plus la différence de couleur entre la racine et les pointes est faible ce qui est le reflet d'une bonne homogénéité de coloration.

c) La chromaticité : C*

La chromaticité dans le système CIE L*, a*, b* est calculée selon l'équation suivante :

$$C^* = \sqrt{a^{*2} + b^{*2}}$$

Plus la valeur de C* est élevée plus la coloration obtenue est chromatique

## II) EXEMPLES COMPARATIFS

### Procédé de coloration

Pour démontrer les performances en termes de coloration des cheveux à partir du procédé selon l'invention, trois exemples comparatifs de procédé en RINCE, NON RINCE MAITRISE et NON RINCE ont été réalisés.

**RINCE** : rinçage intermédiaire des mèches à l'eau du robinet
**NON RINCE** : passage immédiat à la deuxième étape de révélation
**NON RINCE MAITRISE :** essuyage à l'aide d'une serviette absorbante des mèches de cheveux imprégnées de lotion. Une variante consiste également à sécher les cheveux après l'étape d'essuyage ou essorage

| **Exemples** (sur cheveux à 90% de blancs **naturels**) | **Témoin** (cheveux non traités) | **1** comparatif | **2** invention | **3** invention | **4** invention |
|---|---|---|---|---|---|
| Composition A Etape 1 | - | A | A | A | A |
| Etape intermédiaire | - | Rinçage | Essuyé humide | Essuyé séché | Non rincé |
| Composition B Etape 2 | - | B | B | B | B |
| Nuances sur cheveux | - | très léger doré | cuivré très intense | cuivré très intense | cuivré très intense |
| L* | 54,88 | 51,66 | 37,92 | 39,39 | 40,11 |
| a* | 0,87 | 5,54 | 13,38 | 13,35 | 14,51 |
| b* | 12,33 | 21,85 | 25,31 | 26,4 | 28,13 |
| ΔE | - | **11,09** | 24,75 | 24,37 | 25,58 |
| ΔL* | - | -3,22 | -16,96 | -15,49 | -14,77 |
| Δa* | - | 4,67 | 12,51 | 12,48 | 13,63 |
| Δb* | - | 9,53 | 12,98 | 14,07 | 15,81 |

| **Exemples** (sur cheveux à 90% de blancs naturels **permanentés**) | **Témoin** (cheveux non traités) | 5 comparatif | 6 invention | 7 invention | 8 invention |
|---|---|---|---|---|---|
| Composition A Etape 1 | - | A | A | A | A |
| Etape intermédiaire | - | Rinçage | Essuyé humide | Essuyé séché | Non rincé |
| Composition B Etape 2 | - | B | B | B | B |
| Nuances sur cheveux | - | très léger doré-vert | cuivré très intense | cuivré très intense | cuivré très intense |
| L* | 56,59 | 44,83 | 39,18 | 33,96 | 35,02 |
| a* | 0,74 | 2,71 | 15,04 | 14,05 | 12,13 |
| b* | 13,83 | 19,38 | 29,37 | 23,34 | 23,77 |
| ΔE* | - | **13,15** | 27,37 | 27,93 | 26,35 |
| ΔL* | - | -11,76 | -17,41 | -22,63 | -21,57 |
| Δa* | - | 1,98 | 14,31 | 13,31 | 11,4 |
| Δb* | - | 5,55 | 15,54 | 9,51 | 9,94 |

Il apparaît des tableaux ci-dessus que les mèches de cheveux blancs naturels ou permanentés traités avec le procédé qui fait intervenir au moins une étape d'essuyage ou de séchage ou de non rincé maitrisé selon l'invention permet de colorer de façon significativement plus chromatique que la composition selon le comparatif. Par ailleurs les compositions selon l'invention procurent de la couleur aux cheveux beaucoup plus intense que celles obtenues avec les comparatifs (L* plus faible avec les compositions selon l'invention).

La composition suivante a été préparée :

| **Composition A'** | **A'1** |
|---|---|
| Catéchine | 4g |
| Héxylène glycol | 5g |
| Lauryl éther sulfate de sodium (70% en MA) | 3,75 g |
| Chlorure de manganèse tétra-hydraté (soit 0,01% en poids d'équivalent métal $Mn^{2+}$) | 0,036 g |
| Peroxyde d'hydrogène | 1,2 g |
| Acide citrique ou hydroxyde de sodium | qsp pH 5 |
| Eau déminéralisée | qsp 100 g |

La composition **A'** est appliquée sur des mèches de cheveux secs à 90 % de blancs naturels et à 90 % de blancs permanentés avec un rapport de bain de 5 g de formule pour 1 g de cheveux. On laisse ensuite poser pendant 30 minutes à une température de 50 °C.

A l'issu du temps de pose, les cheveux imprégnés de la première composition sont soit essuyés à l'aide d'une serviette de papier absorbant pour enlever l'excédant de formule, soit rincés puis essuyés.

| **Composition B'** | **B'1** |
|---|---|
| Bicarbonate de sodium $NaHCO_3$ | 2,6 g |
| Monoéthanolamine ou Acide citrique | qsp pH 9 |
| Eau déminéralisée | qsp 100 g |

La composition **B'** est ensuite appliquée sur les cheveux avec un rapport de bain de 4g pour 1g de mèche ; le temps de pose est de 10 minutes à température ambiante. Au bout de quelques minutes une coloration très intense apparaît.

Après un rinçage, un shampooing et un séchage au casque des mèches, la coloration des cheveux est évaluée visuellement et lue au spectrocolorimètre Minolta CM3600d,(illuminant D65, angle 10°, composante spéculaire incluse) pour les mesures colorimétriques L*, a*, b*.

| **Exemple** (sur cheveux à 90% de blancs **naturels**) | **9** |
|---|---|
| Composition (A'i) Etape 1 | A'1 |
| Etape intermédiaire | **Non rincé, essuyé** |
| Composition (B'i) Etape 2 | B'1 |
| Nuances sur cheveux | cuivré |
| Chromaticité (C*) | 34,30 |

| **Exemple** (sur cheveux à 90% de blancs naturels **permanentés**) | **9** |
|---|---|
| Composition (A'i) Etape 1 | A'1 |
| Etape intermédiaire | **Non rincé,** essuyé |
| Composition (B'i) Etape 2 | B'1 |
| Nuances sur cheveux | cuivré |
| Chromaticité (C*) | 34,96 |

| **Exemple** (sur cheveux à 90% de blancs **naturels**) | **Comparatif 10** |
|---|---|
| Composition (A'i) Etape 1 | A'1 |
| Etape intermédiaire | **Rincé,** essuyé |
| Composition (B'i) Etape 2 | B'1 |
| Nuances sur cheveux | doré vert |
| Chromaticité (C*) | 30,18 |

| **Exemple** (sur cheveux à 90% de blancs naturels **permanentés**) | **Comparatif 10** |
|---|---|
| Composition (A'i) Etape 1 | A'1 |
| Etape intermédiaire | **Rincé,** essuyé |
| Composition (B'i) Etape 2 | B'1 |
| Nuances sur cheveux | doré vert |
| Chromaticité (C*) | 29,77 |

**Sélectivité**

| | |
|---|---|
| Sélectivité $\Delta E_{sélec}$ (BN/BP) **NON RINCE** + ESSUYAGE (Exemple 9) | 1,08 |
| Sélectivité $\Delta E_{séiec}$ (BN/BP) **RINCE** + ESSUYAGE (Comparatif exemple 10) | 6,08 |

Les fibres kératiniques colorées avec le procédé selon l'invention faisant intervenir une étape de non-rinçage permet d'obtenir des colorations plus homogène, car significativement moins sélective $\Delta E_{sélec}$ que lorsqu'une étape de rinçage est mise en oeuvre avant l'application de l'ingrédient iv) bicarbonate. De plus les procédé selon l'exemple 9 permettent de colorer les cheveux de façon significativement plus chromatique que le comparatif et ce sur fibre kératiniques naturelle ou permanentées.

## Revendications

**1.** Procédé de coloration des fibres kératiniques en plusieurs étapes, consistant à :

a) dans un premier temps traiter lesdites fibres par :

i) un ou plusieurs dérivé(s) d'orthodiphénol(s),
ii) un ou plusieurs sel(s) métallique(s), et
iii) du peroxyde d'hydrogène ou un ou plusieurs système(s) générateur(s) de peroxyde d'hydrogène,

les ingrédients i) à iii) étant appliqués sur lesdites fibres ensemble ou séparément ;
b) dans un deuxième temps lesdites fibres sont essuyées mécaniquement et/ou séchées et/ou non rincées préférentiellement lesdites fibres sont essuyées mécaniquement et/ou séchées et plus particulièrement lesdites fibres sont essuyées mécaniquement ; et
c) dans une troisième étape lesdites fibres sont traitées par iv) un ou plusieurs (bi)carbonate(s)

plus particulièrement juste avant l'étape c) qui met en oeuvre l'ingrédient iv) les fibres sont essuyées mécaniquement, ou

a) dans un premier temps traiter lesdites fibres par :

i) un ou plusieurs dérivé(s) d'orthodiphénol(s), et
ii) un ou plusieurs sel(s) métallique(s),

b) dans un deuxième temps lesdites fibres sont essuyées mécaniquement et/ou séchées et/ou non rincées préférentiellement lesdites fibres sont essuyées mécaniquement et/ou séchées et plus particulièrement lesdites fibres sont essuyées mécaniquement ; et
c) dans une troisième étape lesdites fibres sont traitées par iii) du peroxyde d'hydrogène ou un ou plusieurs système(s) générateur(s) de peroxyde d'hydrogène et iv) un ou plusieurs (bi)carbonate(s) ;

plus particulièrement juste avant l'étape c) qui met en oeuvre l'ingrédient iv) les fibres sont essuyées mécaniquement ; étant entendu que les ingrédients i) et ii) et iii) et iv) peuvent être appliqués sur lesdites fibres ensemble ou séparément.

**2.** Procédé de coloration selon la revendication précédente dans lequel le ou les orthodiphénol(s) choisis parmi les dérivé(s) d'orthodiphénol(s) naturel(s).

**3.** Procédé de coloration selon la revendication 1 ou 2 dans lequel l'ingrédient i) est un orthodiphénol à cycle aromatique choisi parmi le benzène, le naphtalène, le tétrahydronaphtalène, l'indane, l'indène, l'anthracène, le phénanthrène, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine, ledit cycle aromatiques comportant au moins deux groupes hydroxy portés par deux atomes adjacents contigus du cycle aromatique.

4. Procédé de coloration selon une quelconque des revendications précédentes dans lequel l'ingrédient i) est de formule (I) suivante ou l'un de ses oligomères, sous forme salifié ou non :

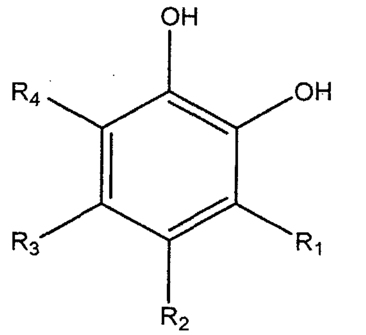

(I)

formule (I) dans laquelle les substituants :

• $R_1$ à $R_4$, identiques ou différents, représentent :

- un atome d'hydrogène,
- un atome halogène,
- un radical hydroxy,
- un radical carboxyle,
- un radical carboxylate d'alkyle ou alcoxycarbonyle,
- un radical amino éventuellement substitué,
- un radical alkyle linéaire ou ramifié éventuellement substitué,
- un radical alcényle linéaire ou ramifié éventuellement substitué,
- un radical cycloalkyle éventuellement substitué,
- un radical alcoxy,
- un radical alcoxyalkyle,
- un radical alcoxyaryle, le groupe aryle pouvant être éventuellement substitué,
- un radical aryle,
- un radical aryle substitué,
- un radical hétérocyclique, saturé ou non, porteur ou non d'une charge cationique ou anionique, éventuellement substitué et/ou éventuellement condensé avec un cycle aromatique, ledit cycle aromatique étant éventuellement substitué,
- un radical contenant un ou plusieurs atomes de silicium,

où deux des substituants portés par deux atome de carbone adjacents $R_1$ - $R_2$, $R_2$ - $R_3$ ou $R_3$ - $R_4$ forment conjointement un cycle saturé ou insaturé, aromatique ou non, substitué ou non, contenant éventuellement un ou plusieurs hétéroatomes et éventuellement condensé avec un ou plusieurs cycles saturés ou insaturés, éventuellement substitué, contenant éventuellement un ou plusieurs hétéroatomes.

5. Procédé de coloration la revendication précédente dans lequel le ou les orthodiphénol(s) sont choisis parmi :

- les flavonols,
- les anthocyanidines,
- les anthocyanines ou les anthocyanes ,
- les orthohydroxybenzoates,
- les flavones,
- les hydroxystilbènes,
- la 3,4-dihydroxyphénylalanine et ses dérivés,
- la 2,3-dihydroxyphénylalanine et ses dérivés,
- la 4,5-dihydroxyphénylalanine et ses dérivés,
- les dihydroxycinnamates,
- les orthopolyhydroxycoumarines,
- les orthopolyhydroxyisocoumarines,
- les orthopolyhydroxycoumarones,

- les orthopolyhydroxyisocoumarones,
- les orthopolyhydroxychalcones,
- les orthopolyhydroxychromones,
- les polyhydroxyquinones,
- les orthohydroxyxanthones,
- le 1,2 dihydroxybenzène et ses dérivés,
- le 1,2,4 trihydroxybenzène et ses dérivés,
- le 1,2,3 trihydroxybenzène et ses dérivés,
- le 2,4,5 trihydroxytoluène et ses dérivés,
- les proanthocyanidines,
- les proanthocyanines,
- l'acide tannique,
- l'acide ellagique,
- et les mélanges des composés précédents.

**6.** Procédé de coloration selon une quelconque des revendications 2 à 5 dans lequel le ou les orthodiphénol(s) naturel(s) i) sont choisis parmi les extraits d'animaux, de bactéries, de champignons, d'algues, de plantes.

**7.** Procédé de coloration selon la revendication précédente dans lequel le ou les orthodiphénol(s) naturel(s) i) sont choisis parmi :

- les extraits de feuilles de thé, les extraits de feuilles de romarin et les extraits de feuilles de maté ;
- les extraits de fruits, tels que les extraits de raisin (en particulier de pépins de raisin ou de marc de raisin), les extraits fèves et/ou cabosses de cacaotier ;
- les extraits de légumes, tels que les extraits de pelures d'oignon ou de salade ;
- les extraits de bois d'arbres, tels que les extraits d'écorce de pin, les extraits de bois de campêche, les extraits de murier des teinturiers dit « bois jaune » ou les extraits d'acacia.

**8.** Procédé de coloration selon une quelconque des revendications précédentes dans lequel le ou les sel(s) métallique(s) contenu(s) ii) sont choisis parmi les oxydes de Mn et Zn.

**9.** Procédé selon la revendication précédente dans lequel les sels de Mn et Zn, sont choisis parmi les halogénures, les sulfates, phosphates, nitrates et perchlorates, les sels d'acides carboxyliques tels que les gluconates ainsi que leurs mélanges.

**10.** Procédé de coloration selon une quelconque des revendications précédentes dans lequel la composition comprenant comme ingrédient iii) contient du peroxyde d'hydrogène ou du peroxyde d'urée.

**11.** Procédé de coloration selon une quelconque des revendications précédentes dans lequel l'ingrédient iv) contient du ou des (bi)carbonate(s) alcalins ou alcalino-terreux.

**12.** Procédé selon une quelconque des revendications précédentes dans lequel lors de l'étape b) les fibres kératiniques sont essuyées mécaniquement une seule fois ou une succession de fois jusqu'à disparition de l'excédent d'ingrédients i) et ii) ou d'ingrédients i), ii) et iii).

**13.** Procédé selon une quelconque des revendications précédentes dans lequel lors de l'étape b) les fibres kératiniques sont séchées thermiquement.

## Patentansprüche

**1.** Verfahren zum Färben von Keratinfasern in mehreren Schritten, das darin besteht, dass man:

a) erstens die Fasern mit:

i) einem öder mehreren ortho-Diphenolderivaten,
ii) einem oder mehreren Metallsalzen und
iii) Wasserstoffperoxid oder einem oder mehreren Wasserstoffperoxid erzeugenden Systemen

behandelt, wobei die Bestandteile i) bis iii) zusammen oder separat auf die Fasern aufgebracht werden;
b) zweitens die Fasern mechanisch abwischt und/oder trocknet und/oder nicht spült, vorzugsweise die Fasern mechanisch abwischt und/oder trocknet und weiter bevorzugt die Fasern mechanisch abwischt; und
c) in einem dritten Schritt die Fasern mit iv) einem oder mehreren (Hydrogen)carbonaten behandelt,

weiter bevorzugt ummittelbar vor Schritt c), bei dem der Bestandteil iv) eingesetzt wird, die Fasern mechanisch abwischt,
oder

a) erstens die Fasern mit:

i) einem oder mehreren ortho-Diphenolderivaten und
ii) einem oder mehreren Metallsalzen behandelt,

b) zweitens die Fasern mechanisch abwischt und/oder trocknet und/oder nicht spült, vorzugsweise die Fasern mechanisch abwischt und/oder trocknet und weiter bevorzugt die Fasern mechanisch abwischt; und
c) in einem dritten Schritt die Fasern mit iii) Wasserstoffperoxid oder einem oder mehreren Wasserstoffperoxid erzeugenden Systemen und iv) einem oder mehreren (Hydrogen)carbonaten behandelt,

weiter bevorzugt ummittelbar vor Schritt c), bei dem der Bestandteil iv) eingesetzt wird, die Fasern mechanisch abwischt,
mit der Maßgabe, dass die Bestandteile i) und ii) und iii) und iv) zusammen oder separat auf die Fasern aufgebracht werden können.

**2.** Färbeverfahren nach dem vorhergehenden Anspruch, bei dem man das ortho-Diphenol bzw. die ortho-Diphenole aus natürlichen ortho-Diphenolderivaten auswählt.

**3.** Färbeverfahren nach Anspruch 1 oder 2, bei dem es sich bei dem Bestandteil i) um ein ortho-Diphenol mit einem aromatischen Ring, der aus Benzol, Naphthalin, Tetrahydronaphthalin, Indan, Inden, Anthracen, Phenanthren, Isoindol, Indolin, Isoindolin, Benzofuran, Dihydrobenzofuran, Chroman, Isochroman, Chromen, Isochromen, Chinolin, Tetrahydrochinolin und Isochinolin ausgewählt ist, handelt, wobei der aromatische Ring mindestens zwei Hydroxygruppen an zwei aneinandergrenzenden benachbarten Atomen des aromatischen Rings umfasst.

**4.** Färbeverfahren nach einem der vorhergehenden Ansprüche, bei dem der Bestandteil i) die folgende Formel (I) aufweist oder ein Oligomer davon ist, gegebenenfalls in versalzter Form:

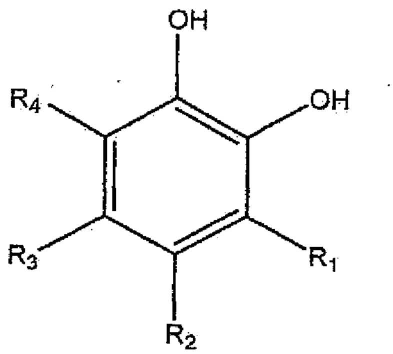

(I)

wobei in Formel (I) die Substituenten:

• $R_1$ bis $R_4$ gleich oder verschieden sind und für:

- ein Wasserstoffatom,
- ein Halogenatom,
- einen Hydroxylrest,
- einen Carboxylrest,
- einen Alkylcarboxylat- oder Alkoxycarbonylrest,

- einen gegebenenfalls substituierten Aminorest,
- einen gegebenenfalls substituierten linearen oder verzweigten Alkylrest,
- einen gegebenenfalls substituierten linearen oder verzweigten Alkenylrest,
- einen gegebenenfalls substituierten Cycloalkylrest,
- einen Alkoxyrest,
- einen Alkoxyalkylrest,
- einen Alkoxyarylrest, wobei die Arylgruppe gegebenenfalls substituiert sein kann,
- einen Arylrest,
- einen substituierten Arylrest,
- einen gesättigten oder ungesättigen heterocyclischen Rest, der gegebenenfalls eine kationische oder anionische Ladung trägt, gegebenenfalls substituiert ist und/oder gegebenenfalls mit einem aromatischen Ring kondensiert ist, wobei der aromatische Ring gegebenenfalls substituiert ist,
- einen Rest, der ein oder mehrere Siliciumatome enthält,

stehen,
wobei zwei der Substitutenten an zwei benachbarten Köhlenstoffatomen $R_1$-$R_2$, $R_2$-$R_3$ oder $R_3$-$R_4$ zusammen einen gesättigten oder ungesättigten, aromatischen oder nichtaromatischen, substituierten oder unsubstituierten Ring bilden, der gegebenenfalls ein oder mehrere Heteroatome enthält und gegebenenfalls mit einem oder mehreren gesättigten oder ungesättigten, gegebenenfalls substituierten Ringen, die gegebenenfalls ein oder mehrere Heteroatome enthalten, kondensiert ist.

**5.** Färbeverfahren nach dem vorhergehenden Anspruch, bei dem man das ortho-Diphenol bzw. die ortho-Diphenole aus:

- Flavonolen,
- Anthocyanidinen,
- Anthocyaninen oder Anthocyanen,
- ortho-Hydroxybenzoaten,
- Flavonen,
- Hydroxystilbenen,
- 3,4-Dihydroxyphenylalanin und Derivaten davon,
- 2,3-Dihydroxyphenylalanin und Derivaten davon,
- 4,5-Dihydroxyphenylalanin und Derivaten davon,
- Dihydroxycinnamaten,
- ortho-Polyhydroxycumarinen,
- ortho-Polyhydroxyisocumarinen,
- ortho-Polyhydroxycumaronen,
- ortho-Polyhydroxyisocumaronen,
- ortho-Polyhydroxychalconen,
- ortho-Polyhydroxychromonen,
- Polyhydroxychinonen,
- ortho-Hydroxyxanthonen,
- 1,2-Dihydroxybenzol und Derivaten davon,
- 1,2,4-Trihydroxybenzol und Derivaten davon,
- 1,2,3-Trihydroxybenzol und Derivaten davon,
- 2,4,5-Trihydroxytoluol und Derivaten davon,
- Proanthocyanidinen,
- Proanthocyaninen,
- Tanninsäure,
- Ellaginsäure
- und Mischungen der vorstehenden Verbindungen auswählt.

**6.** Färbeverfahren nach einem der Ansprüche 2 bis 5, bei dem man das natürliche ortho-Diphenol bzw. die natürlichen ortho-Diphenole aus Tier-, Bakterien-, Pilz-, Algen- und Pflanzenextrakten auswählt.

**7.** Färbeverfahren nach dem vorhergehenden Anspruch, bei dem man das natürliche ortho-Diphenol bzw. die natürlichen ortho-Diphenole aus:

- Teeblattextrakten, Rosmarinblattextrakten und Mateblattextrakten;
- Fruchtextrakten, wie Traubenextrakten (insbesondere Traubenkern- oder Traubenmark-extrakten) oder Kakaobohnen- und/oder Kakaoschotenextrakten;
- Gemüseextrakten, wie Zwiebelschalen- oder Salatextrakten;
- Baumholzextrakten, wie Kieferrindenextrakten, Kampeschenholzextrakten, Extrakten des als "Gelbholz" bezeichneten Färbermaulbeerbaums oder Akazienextrakten;

auswählt.

**8.** Färbeverfahren nach einem der vorhergehenden Ansprüche, bei dem man das bzw. die enthaltenen Metallsalze ii) aus Mn- und Zn-Oxiden auswählt.

**9.** Verfahren nach dem vorhergehenden Anspruch, bei dem man die Mn- und Zn-Salze aus Halogeniden, Sulfaten, Phosphaten, Nitraten und Perchlorate Carbonsäuresalzen wie Gluconaten sowie Mischungen davon auswählt.

**10.** Färbeverfahren nach einem der vorhergehenden Ansprüche, bei dem die Zusammensetzung umfassend als Bestandteil iii) Wasserstoffperoxid oder Harnstoffperoxid umfasst.

**11.** Färbeverfahren nach einem der vorhergehenden Ansprüche, bei dem der Bestandteil iv) Alkalimetall- oder Erdalkalimetall(hydrogen)carbonate enthält.

**12.** Verfahren nach einem der vorhergehenden Ansprüche, bei dem man bei Schritt b) die Keratinfasern einmal oder mehrmals mechanisch abwischt, bis der Überschuss der Bestandteile i) und ii) bzw. der Bestandteile i), ii) und iii) verschwunden ist.

**13.** Verfahren nach einem der vorhergehenden Ansprüche, bei dem man bei Schritt b) die Keratinfasern thermisch trocknet.

## Claims

**1.** Multistage method for dyeing keratinous fibers, which consists in:

a) in a first step, treating said fibers with:

i) one or more ortho-diphenol derivative(s),
ii) one or more metal salt(s), and
iii) hydrogen peroxide or one or more system(s) which generate(s) hydrogen peroxide,

the ingredients i) to iii) being applied to said fibers together or separately;
b) in a second step, said fibers are wiped mechanically and/or dried and/or not rinsed, preferably said fibers are wiped mechanically and/or dried and more particularly said fibers are wiped mechanically; and
c) in a third stage, said fibers are treated with iv) one or more (bi)carbonate(s);
more particularly, just before stage c), which uses the ingredient iv), the fibers are wiped mechanically;

or:

a) in a first step, treating said fibers with:

i) one or more ortho-diphenol derivative(s), and
ii) one or more metal salt(s),

b) in a second step, said fibers are wiped mechanically and/or dried and/or not rinsed, preferably said fibers are wiped mechanically and/or dried and more particularly said fibers are wiped mechanically; and
c) in a third stage, said fibers are treated with iii) hydrogen peroxide or one or more system(s) which generate(s) hydrogen peroxide and iv) one or more (bi)carbonate(s);
more particularly, just before stage c), which uses the ingredient iv), the fibers are wiped mechanically;

it being understood that the ingredients i) and ii) and iii) and iv) can be applied to said fibers together or separately.

2. Dyeing method according to the preceding claim, in which the ortho-diphenol(s) are chosen from natural ortho-diphenol derivative(s).

3. Dyeing method according to Claim 1 or 2, in which the ingredient i) is an ortho-diphenol comprising an aromatic ring chosen from benzene, naphthalene, tetrahydronaphthalene, indane, indene, anthracene, phenanthrene, isoindole, indoline, isoindoline, benzofuran, dihydrobenzofuran, chromane, isochromane, chromene, isochromene, quinoline, tetrahydroquinoline and isoquinoline, said aromatic ring comprising at least two hydroxyl groups carried by two contiguous adjacent atoms of the aromatic ring.

4. Dyeing method according to any one of the preceding claims, in which the ingredient i) is of following formula (I) or one of its oligomers, in or not in the salified form:

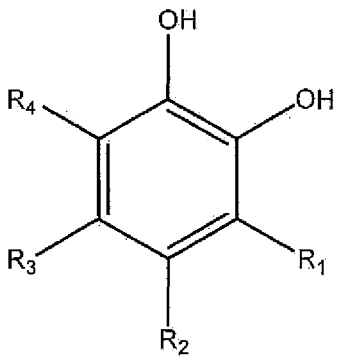

(I)

in which formula (I) the substituents:

- $R_1$ to $R_4$, which are identical or different, represent:
  - a hydrogen atom,
  - a halogen atom,
  - a hydroxyl radical,
  - a carboxyl radical,
  - an alkyl carboxylate or alkoxycarbonyl radical,
  - an optionally substituted amino radical,
  - an optionally substituted linear or branched alkyl radical,
  - an optionally substituted linear or branched alkenyl radical,
  - an optionally substituted cycloalkyl radical,
  - an alkoxy radical,
  - an alkoxyalkyl radical,
  - an alkoxyaryl radical, it being possible for the aryl group to be optionally substituted,
  - an aryl radical,
  - a substituted aryl radical,
  - a saturated or unsaturated heterocyclic radical which does or does not carry a cationic or anionic charge, which is optionally substituted and/or which is optionally fused with an aromatic ring, said aromatic ring being optionally substituted,
  - a radical comprising one or more silicon atoms;

- or two of the substituents carried by two adjacent carbon atoms $R_1$-$R_2$, $R_2$-$R_3$ or $R_3$-$R_4$ jointly form a saturated or unsatuarated, aromatic or nonaromatic and substituted or unsubstituted ring optionally comprising one or more heteroatoms and optionally fused with one or more saturated or unsaturated and optionally substituted rings optionally comprising one or more heteroatoms.

5. Dyeing method according to the preceding claim, in which the ortho-diphehol(s) are chosen from:

- flavonols,

- anthocyanidins,
- anthocyanins or anthocyans,
- orthohydroxybenzoates,
- flavones,
- hydroxystilbenes,
- 3,4-dihydroxyphenylalanine and its derivatives,
- 2,3-dihydroxyphenylalanine and its derivatives,
- 4, 5-dihydroxyphenylalanine and its derivatives,
- dihydroxycinnamates,
- orthopolyhydroxycoumarins,
- orthopolyhydroxyisocoumarins,
- orthopolyhydroxycoumarones,
- orthopolyhydroxyisocoumarones,
- orthopolyhydroxychalcones,
- orthopolyhydroxychromones,
- orthopolyhydroxyquinones,
- orthohydroxyxanthones,
- 1,2-dihydroxybenzene and its derivatives,
- 1,2,4-trihydroxybenzene and its derivatives,
- 1,2,3-trihydroxybenzene and its derivatives,
- 2,4,5-trihydroxytoluene and its derivatives,
- proanthocyanidins,
- proanthocyanins,
- tannic acid,
- ellagic acid,
- and the mixtures of the above compounds.

**6.** Dyeing method according to any one of Claims 2 to 5, in which the natural ortho-diphenol(s) i) are chosen from extracts of animals, bacteria, fungi, algae or plants.

**7.** Dyeing method according to the preceding claim, in which the natural ortho-diphenol(s) i) are chosen from:

- extracts of tea leaves, extracts of rosemary leaves and extracts of mate leaves;
- extracts of fruits, such as extracts of grape (in particular of grape seeds or of grape marc), or extracts of cocoa beans and/or pods;
- extracts of vegetables, such as extracts of onion skins or of salad;
- extracts of tree wood, such as extracts of pine bark, extracts of logwood, extracts of dyer’s mulberry ("yellow wood") or extracts of acacia.

**8.** Dyeing method according to any one of the preceding claims, in which the metal salt(s) ii) present are chosen from manganese and zinc oxides.

**9.** Method according to the preceding claim, in which the manganese and zinc salts are chosen from halides, sulfates, phosphates, nitrates and perchlorates, salts of carboxylic acids, such as gluconates, and also their mixtures.

**10.** Dyeing method according to any one of the preceding claims, in which the composition comprising as ingredient iii) comprises hydrogen peroxide or urea hydrogen peroxide.

**11.** Dyeing method according to any one of the preceding claims, in which the ingredient iv) comprises alkali metal or alkaline earth metal (bi)carbonate(s).

**12.** Method according to any one of the preceding claims, in which, during stage b), the keratinous fibers are wiped mechanically once or a series of times until the excess ingredients i) and ii) or ingredients i), ii) and iii) have disappeared.

**13.** Method according to any one of the preceding claims, in which, during stage b), the keratinous fibers are dried using heat.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2814943
- FR 2814945
- FR 2814946
- FR 2814947
- US 5008093 A
- US 3376110 A
- US 5183901 A